# EUROPÄISCHE PATENTANMELDUNG

(11) **EP 1 958 932 A1**
(43) Veröffentlichungstag der Anmeldung: **20.08.2008**
(21) Anmeldenummer: 07003275.0
(22) Anmeldetag: 16.02.2007
(51) Int. Cl.: C07C 69/34, C10M 129/72, C10M 105/36

(54) **Ester und deren Mischungen sowie deren Verwendung als Schmiermittel oder in Hydraulikölen**

(71) Anmelder: Cognis IP Management GmbH, 40589 Düsseldorf (DE)
(72) Erfinder: Busch, Dr. Stefan, DE-46047 Oberhausen (DE); Röder, Jürgen, DE - 40589 Düsseldorf (DE)

(57) **Zusammenfassung**

Beschrieben werden Estermischungen enthaltend (a) mindestens einen Ester dessen Alkoholteil ausgewählt ist aus verzweigten Monoalkoholen mit mindestens 4 C-Atomen und dessen Säureteil ausgewählt ist aus verzweigten oder linearen Carbonsäuren mit 2 bis 22 C-Atomen und 2 bis 4 Carboxylgruppen, und (b) mindestens einen Ester einer Dimerfettsäure mit einem verzweigten Monoalkohol mit mindestens 4 C-Atomen. Diese Estermischungen eignen sich insbesondere als Schmierstoffe oder in Hydraulikflüssigkeiten. Geeignet sind auch die Ester des Typs (b) alleine.

## Beschreibung

Die vorliegende Erfindung betrifft Ester und Estermischungen von bestimmten Carbonsäureestern und deren Verwendung als Schmierstoffe oder in Hydraulikölen.

Carbonsäureester sind seit langem bekannte organische Verbindungen, die auch breite Anwendung in Schmiermitteln oder in Hydraulikanwendungen, beispielsweise als Hydrauliköl gefunden haben. Solche Esteröle sollten möglichst niedrige Pourpoints (< -40° C), einen hohen Viskositätsindex (> 140), eine niedrige Flüssigflüchtigkeit und einen hohen Flammpunkt (> 250° C) und gleichzeitig eine hohe Thermo- bzw. Oxidationsstabilität aufweisen. Weiterhin sollen sie gut biologisch abbaubar sein und vorzugsweise aus Rohstoffquellen stammen, die durch nachwachsende Rohstoffe bedient werden können.

Gängige Ester, die in diesen Anwendungsbereichen Verwendung finden, basieren auf Reaktionsprodukten von Polyolen, beispielsweise Trimethylolpropan oder Pentaerytrithol mit Fettsäuren, wie beispielsweise Ölsäure oder Mischungen unterschiedlich langer gesättigter Fettsäuren. Häufig werden auch Fettsäuremischungen mit den Polyolen zur Reaktion gebracht, um auf diese Weise bestimmte anwendungstechnisch relevante Eigenschaften wie die Viskosität oder Tieftemperaturverhalten zu optimieren.
Aus der EP 0 188 535 sind beispielsweise flüssige Ester, die 52 bis 150 Kohlenstoffatome enthalten, bekannt, wobei hier als Alkoholkomponente Guerbetalkohole und zum Teil polymere Fettsäuren nebeneinander zur Herstellung von Estern eingesetzt werden können.
Die DE 10 2004 034 202 A1 beschreibt Estermischungen, bei denen primäre verzweigte Alkohole mit Di-, Tri- oder Tetracarbonsäuren zur Reaktion gebracht werden. Es werden soweit Ester hergestellt aus Alkoholen und monomeren Polycarbonsäuren, also Carbonsäuren, die mehr als eine Carboxylfunktion in den Molekülen enthalten, beschrieben. Die so hergestellten Ester eignen sich als Motoren-, Hydraulik-, Kurbelwellen- oder Kompressoröle und können generell als Schmierstoffe oder Hydraulikflüssigkeit Verwendung finden. Die Lehre der DE 10 2004 034 202 betrifft aber hauptsächlich solche Estermischungen deren kinematische Viskosität bei 40 °C bei kleiner 50 mm²/s liegen soll. Es werden daher in den Beispielen auch nur solche Estermischungen offenbart, deren kinematische Viskosität bei 40 °C maximal 31,5 mm²/s beträgt.

Es besteht allerdings weiterhin ein Bedarf nach verbesserten Schmierstoffen auf Esterbasis, wobei diese Schmierstoffe eine erhöhte kinematische Viskosität, vorzugsweise von mehr als 40 mm²/s besser mehr als 50 mm²/s, jeweils bei 40 °C aufweisen sollen. Gleichzeitig sollen solche Schmiermittel eine gute Tieftemperaturverträglichkeit aufweisen, also vorzugsweise einen niedrigen Pourpoint zeigen und trotzdem einen hohen Viskositätsindex, insbesondere von mehr als 120 und vorzugsweise mehr als 140 aufweisen. Bisher wurden solche Anforderungen durch Mischung an sich bekannter Ester mit diversen Additiven erreicht. Diese Additive können aber zu Problemen bei der Scherstabilität, der Dichtungsverträglichkeit, dem Demulgiervermögen oder der Hydrolyseneigung führen oder nicht ausreichend biologisch abbaubar sein. Die Aufgabe der vorliegenden Erfindung bestand daher darin, Ester bereit zu stellen, die diese Nachteile vermeiden.

Es wurde gefunden, dass bestimmte Ester auf Basis von Oligomeren von Monocarbonsäuren und Estermischungen, die solche Ester enthalten, die gewünschten Eigenschaften aufweisen.

Ein erster Gegenstand der Erfindung betrifft deshalb Ester, die als Alkoholteil (i) einen verzweigten einwertigen Alkohol mit mindestens 4 C-Atomen und als Säureteil (ii) Oligomere aus Monocarbonsäuren mit 2 bis 22 C-Atomen und vorzugsweise 6 bis 22 C-Atomen enthalten.

### Alkoholkomponente (i)

Als Alkoholkomponente (i) sind vorzugsweise verzweigte primäre Alkohole - also solche, die nur eine freie Hydroxylfunktion im Molekül enthalten, mit 4 bis 44 C-Atomen, und weiterhin vorzugsweise 6 bis 24 C-Atomen und besonders bevorzugt mit 14 bis 24 C-Atomen ausgewählt.

Besonders bevorzugt ist dabei als verzweigter primärer Alkohol (i) ein Guerbet-Alkohol mit 4 bis 44, vorzugsweise 6 bis 24, insbesondere 14 bis 24 und besonders bevorzugt mit 18 bis 22 C-Atomen ausgewählt. Während prinzipiell auch andere verzweigte Alkohole geeignet sind, im Sinne der vorliegenden technischen Lehre eingesetzt zu werden, handelt es sich bei den Guerbetalkoholen um besonders bevorzugte Substrate zur Herstellung der erfindungsgemäßen Ester.
Guerbetalkohole werden z.B. durch Selbstkondensation primärer Alkohole unter dem Einfluss von Natrium oder Kupferkatalysatoren bei 200° C oder mehr und erhöhtem Druck hergestellt. Typische Guerbetalkohole sind beispielsweise 2-Methyl-1-Penthynol, 2-Ethyl-1-Hexanol, 2-Propyl-1-Heptanol, 2-Butyl-1-Octanol, 2-Penthyl-1-Nonamol, 2-Hexyl-1-Decanol, 2-Hepthyl-1-Indicanol, 2-Octyl-1-Dodecanol, 2-Monyl-1-Tridecanol, 2-Decyl-1-Tetradecanol, 2-Umdecyl-1-Pentadecanol, 2-Dodecyl-1-Hexadecanol, 2-Tridecyl-1-Heptadecanol, 2-Tetradecyl-1-Octadecanol, 2-Pentadecyl-1-Nonadecanol, 2-Hexadecyl-1-Eicosanol, 2-Heptadecyl-1-Heneicosanol, 2-Octadecyl-1-Docosanol, 2-Nonaedecyl-1-Tricosanol, 2-Eicosyl-1-Tetracosanol.

### Säurekomponente (ii)

Die erfindungsgemäßen Ester enthalten als Säurekomponente (ii) Oligomere von Monocarbonsäuren. Als Monocarbonsäuren werden solche organischen Verbindungen verstanden, die nur eine frei Carboxylfunktion -COOX im Molekül enthalten, wobei X für ein Wasserstoffatom oder für ein ein- oder mehrwertiges Anion stehen kann. Diese Oligomere müssen mindestens zwei und maximal 5 Monomerbestandteile enthalten. Geeignete Monomere sind insbesondere Fettsäuren mit 6 bis 22 C-Atomen.

Unter Fettsäuren sind dabei zunächst alle Carbonsäuren der Formel (I) zu verstehen

R¹CO-OH (I)

in der R¹CO für einen linearen oder verzweigten Acylrest mit 6 bis 22 Kohlenstoffatomen und 0 und/oder 1, 2 oder 3 Doppelbindungen steht. Typische Beispiele sind Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelenschen Oxosynthese oder der Dimerisierung von ungesättigten Fettsäuren anfallen.

Zur Oligomerisierung, d.h. der Reaktion mehrere der Monocarbonsäuren miteinander, eignen sich aber vorzugsweise ungesättigte Fettsäuren der oben beschriebenen Art, hiervon besonders Ölsäure und Linolsäure, die als Mischung beispielsweise in Tallölfettsäure vorkommen, welche daher auch ein wichtigen Rohstoff zur Herstellung von Polymersäuren darstellt.

Das Rohprodukt der Oligomersierung von C₁₈-Fettsäuren enthält neben linearen und verzweigten Monocarbonsäuren, den Monomerfettsäuren, Dimerfettsäure mit 36 C-Atomen, Trimerfettsäure mit 54 C-Atomen sowie höhere Polymerfettsäuren in geringeren Mengen. Der jeweilige Anteil der Homologen hängt von den eingesetzten Rohstoffen sowie den Oligomerisierungsbedingungen ab. Das Rohprodukt wird üblicherweise durch Destillation oder Extraktion fraktioniert, um beispielsweise hoch dimerfettsäurehaltige Schnitte zu erhalten.
Wegen ihrer guten Verfügbarkeit werden kommerziell erhältliche Dimerfettsäuren als Komponente (ii) bevorzugt. Dabei sind solche Dimerfettsäuren (ii) bevorzugt die auf Basis von Ölsäure, Ricinfettsäure und Tallölfettsäure ausgewählt sind, wobei Dimerfettsäuren auf Basis von Tallölfettsäuren besonders bevorzugt sind.
Die technischen Dimerfettsäuren enthalten je nach Typ oder Qualität neben der eigentlichen Dimersäure weitere Homologe, hauptsächlich Trimerfettsäure. Hochreine Dimerfettsäuren bestehen zu > 97% aus Dicarbonsäure. Bevorzugt sind technische Dimerfettsäuren, die Dimerfettsäuren mit 36 C-Atomen als Hauptkomponente enthalten. Die Säurezahl von hochreinen Dimerfettsäuren liegt in der Regel zwischen 195 und 198 mg KOH/g.

Die erfindungsgemäßen Ester weisen vorzugsweise eine kinematische Viskosität bei 40°C von größer 50 mm²/s bis 160 mm²/s und vorzugsweise von größer 100 mm²/s bis 160 mm²/s auf.

Ein weiterer Gegenstand der vorliegenden Anmeldung betrifft die Verwendung der beschriebenen Ester zur Viskositätserhöhung, insbesondere bei Schmierstoffen oder Hydraulikölen, und hier vorzugsweise bei solchen Mitteln, die andere Ester als Komponenten enthalten können.

Ein weiterer Gegenstand der vorliegenden Erfindung sind Estermischungen enthaltend
(a) mindestens einen der oben beschriebenen Ester bestehend aus den Komponenten (i) und (ii) sowie
(b) mindestens einen Ester, dessen Alkoholteil (iii) ausgewählt ist aus verzweigten primären Alkoholen mit mindestens 4 C-Atomen und dessen Säureteil (iv) ausgewählt ist aus Dicarbonsäuren.

Dabei gilt für den Ester der Komponente (b), dass dessen Alkoholteil (iii) vorzugsweise ausgewählt ist aus der Gruppe der in der Beschreibung der Komponente (ii) genannten Alkohole.

Der Säureteil (iv) der Esterkomponente (b) ist vorzugsweise ausgewählt aus verzweigten oder unverzweigten gesättigten oder ungesättigten Dicarbonsäuren, vorzugsweise aus linearen Dicarbonsäuren. Dicarbonsäuren enthalten im Molekül mindestens zwei Carboxylfuktionen -COOX gemäß der obigen Beschreibung, sind aber nicht durch Oligomerisierung erhältlich und somit zu unterscheiden von der Säurekomponente (ii). Die Säuren des Typs (iv) enthalten vorzugsweise 2 bis 18 C-Atome, und insbesondere 4 bis 16 C-Atome.
Besonders geeignete Dicarbonsäuren des Typs (iv) sind ausgewählt aus der Gruppe Oxalsäure, Malonsäure, Bernsteinsäure, Glutarsäure, Adipinsäure, Pimelinsäure, Korksäure, Azelainsäure, Sebazinsäure, Maleinsäure, Marsäure, aber vorzugsweise aus Adipinsäure, Azelainsäure und Sebazinsäure. Ebenso ist es möglich, Mischungen der oben beschriebenen Säuren einzusetzen.

In einer besonderen Ausführungsform enthalten die Estermischungen solche Esterkomponenten (a) und (b), bei denen die Alkoholkomponenten (i) und (iii) der Ester jeweils aus gleichen Alkoholen bzw. Alkoholmischungen ausgewählt worden sind. In einer weiteren Ausführungsform handelt es sich um unterschiedlich strukturierte Alkohole für die Estermischung der Komponente (a) bzw. (b).

Die erfindungsgemäßen Ester aber auch die Estermischungen weisen vorzugsweise einen Viskositätsindex (gemäß DIN ISO 2909) von vorzugsweise mind. 140 auf, insbesondere aber einen Viskositätsindex der >/= 150 und insbesondere >/= 160 ist. Die Ester oder die Estermischungen weisen außerdem vorzugsweise einen Pourpoint (gemäß DIN ISO 3016) von max. -35 °C auf, vorzugsweise aber sind solche Estermischungen bevorzugt, die max. -40 °C als Pourpoint und insbesondere max. - 45° C aufweisen können.

Die erfindungsgemäßen Ester- bzw. Estermischungen zeichnen sich weiterhin durch eine erhöhte Viskosität aus, wobei hier vorzugsweise eine kinematische Viskosität (gemäß DIN 51519) bei 40 °C von größer 50 mm²/s, vorzugsweise mind. 100 mm²/s und insbesondere mind. 125 mm²/s erreicht werden kann, wobei Ester bzw. Estermischungen auch erfindungsgemäß kinematische Viskositäten bei 40 °C von 150 bis 160 mm²/s als Obergrenze aufweisen können. Bevorzugt im Sinne der vorliegenden technischen Lehre sind außerdem solche Ester bzw. Estermischungen, die eine kinematische Viskosität bei 40 °C im Bereich von mehr als 50 mm²/s bis 160 mm²/s und insbesondere im Bereich von größer 100 mm²/s bis 160 mm²/s aufweisen.

Ester und Estermischungen im Sinne der Erfindung fallen vorzugsweise unter die ISO Viskositätsklassen (gemäß DIN 51519) ISO VG 46 bis ISO VG 150 und insbesondere ISO VG 68 bis ISO VG 150.

Ein besonderer Aspekt der vorliegenden Erfindung besteht darin, dass durch Auswahl geeigneter Verhältnisse zwischen linearen Fettsäuren im Vergleich zu den Oligomeren der Fettsäuren die gewünschte Viskosität der Ester- bzw. der Estermischungen variabel einstellbar ist. Auch die Alkoholkomponente kann ggf. variiert werden.

Besonders bevorzugte Ester oder Estermischungen zeigen vorzugsweise einen Viskositätsindex von mind. 150, einen Pourpoint von mind. -35 °C und eine kinematische Viskosität bei 40° C von mind. 50 mm²/s. Bevorzugt sind in diesem Zusammenhang außerdem solche Ester oder Estermischungen, die noch niedrigere Pourpoints als -35°C erreichen.

Die Herstellung der Ester erfolgt in dem Fachmann bekannter Art und Weise durch Umsetzung der Alkohole mit den Säuren in Gegenwart geeigneter z.B. saurer oder alkalischer Katalysatoren und ggf. erhöhter Temperatur und/oder verringertem Druck. Die Herstellung der Estermischung erfolgt ebenfalls in an sich dem Fachmann bekannter Art und Weise und vorzugsweise dadurch, dass die Säure- und Alkoholkomponenten vermischt und in Gegenwart eines Katalysators ggf. bei erhöhter Temperatur und/oder verringertem Druck zur Reaktion gebracht werden. Es ist so möglich, in einer Eintopfreaktion eine erfindungsgemäße Estermischung direkt herzustellen. Es ist allerdings auch möglich und besonders bevorzugt, zunächst die beiden Komponenten (a) und (b) einer Estermischung getrennt herzustellen und anschließend zu vermischen.

Die Ester wie die Estermischung im Sinne der vorliegenden Erfindung eignen sich vorzugsweise zur Verwendung als Schmierstoffe bzw. Schmierstoffkomponente oder als Hydrauliköl bzw. als Bestandteil eines Hydrauliköls. Dabei können die Ester bzw. die Mischungen sowohl in untergeordneten Mengen (0,1 bis kleiner als 50 Gew.-%), quasi als Additive aber auch als Hauptbestanteile (50 Gew.-% bis max. 100 Gew.-%) solcher Öle vorliegen.

Bei den Schmierstoffen seien exemplarisch genannt Motorenöle, Kurbelwellenöle, Kompressoröle, aber auch Kältemaschinenöle, Kohlenwasserstoffkompressoröle, Kühlschmierstoffe, Tieftemperaturschmieröle und Fette, Hochtemperaturschmieröle und Fette, Drahtseilschmierstoffe, Textilmaschinenöle, Kältemaschinenöle, Fluggetriebeöle, Spindelöle, Spinnöle, Traktionsfluide, Getriebeöle, Kunststoffgetriebeöle, Kfz.-Getriebeöle, LKW-Getriebeöle, Industriegetriebeöle, Isolieröle, Instrumentenöle, Lagerschmieröle, Bremsflüssigkeiten, Transmissionsflüssigkeiten, Stoßdämpferöle, Wärmeträgeröle, Transformatorenöle, Fette, Kettenöle, Minimalmengenschmierstoffe, Öle zum Warm- und Kaltumformen, Öle für öl- oder wasserbasierte Metallbearbeitungsflüssigkeiten oder Bohrspülmittel für den Erdreichaufschluss. Weiterhin können die Estermischungen Anwendung finden als Grundöl in Hydraulikölen.

### Beispiele

### 1) Herstellung eines Esters aus Guerbet-Alkohol und einer Dimerfettsäure.

321,7 g (0,55 mol) einer C₁₈-Dimerfettsäure (0,55 mol - Empol 1026 - Fa. Cognis) wurden unter Stickstoffatmosphäre mit 374 g (1,2 mol) eines C₂₀-Guerbet-Alkohols (Eutanol G - Fa. Cognis) verrührt und mit 0,21 g Zinnoxalat (Fascat 2001) versetzt. Danach wurde das Gemisch ca. 3 Stunden auf 240 °C erhitzt. Die Reaktion wurde beendet nachdem die Säurezahl (SZ) des Reaktionsgemisches unter 0,5 mg KOH/g gefallen war. Das Rohprodukt wurde ca. 1 Stunde lang mit Tonsil bei 90 °C gebleicht und das gebleichte Produkt bei verringertem Druck bei 110 °C getrocknet. Das Endprodukt zeigte eine Säurezahl von 3,6 mg KOH/g, eine OH-Zahl von 5,8 mg KOH/g. Die kinematische Viskosität bei 40 °C betrug 150 mm²/s. Der Viskositätsindex betrug 151. Der Pourpoint wurde mit < - 47 °C gemessen.

### 2) Herstellung einer Estermischung durch Direktsynthese

261,1 g C₁₈-Dimerfettsäure (0,45 mol - Empol 1026 - Fa. Cognis) wurden unter Stickstoffatmosphäre mit 188,2 g (1,0 mol) Azelainsäure und 989,1 g (3,17 mol) eines C₂₀-Guerbet-Alkohols (Eutanol G - Fa. Cognis) verrührt und mit 0,4 g Zinnoxalat (Fascat 2001) versetzt. Die weitere Reaktion erfolgte, wie im Beispiel 1 beschrieben. Das Endprodukt zeigte eine Säurezahl von 0,3 mg KOH/g, eine OH-Zahl von 3,5 mg KOH/g. Die kinematische Viskosität bei 40 °C betrug 69 mm²/s. Der Viskositätsindex betrug 167. Der Pourpoint wurde mit < -41 °C gemessen.

### 3) Estermischung

Es wurden 40 g eines Esters gemäß Beispiel 1 mit 60 g eines Esters aus C₂₀-Guerbetalkohl mit Azelainsäure vermischt. Diese Mischung zeigte eine kinematische Viskosität bei 40 °C von 68 mm²/s. Der Viskositätsindex betrug 164. Der Pourpoint wurde mit -40 °C gemessen.

## Patentansprüche

1. Ester, enthaltend als Alkoholteil (i) einen verzweigten einwertigen Alkohol mit mindestens 4 C-Atomen und als Säureteil (ii) Oligomere aus Monocarbonsäuren mit 2 bis 22 C-Atomen und vorzugsweise 6 bis 22 C-Atomen.

2. Ester nach Anspruch 1, **dadurch gekennzeichnet, dass** als Komponente (i) verzweigten primäre Alkoholen mit 4 bis 44 C-Atomen, vorzugsweise 6 bis 24 C-Atomen und besonders bevorzugt mit 14 bis 24 C-Atomen ausgewählt sind.

3. Ester nach mindestens einem der Ansprüche 1 bis 2, **dadurch gekennzeichnet, dass** als verzweigter primärer Alkohol (i) ein Guerbet-Alkohol mit 4 bis 44, vorzugsweise 6 bis 24, insbesondere 14 bis 24 und besonders bevorzugt mit 18 bis 22 C-Atomen ausgewählt ist.

4. Ester nach mindestens einem der Ansprüche 1 bis 3, **dadurch gekennzeichnet, dass** als Komponente (ii) solche oligomeren Monocarbonsäuren ausgewählt sind, die eine technische Dimerfettsäuren mit 36 C-Atomen als Hauptkomponente enthalten.

5. Ester nach mindestens einem der Ansprüche 1 bis 4, **dadurch gekennzeichnet, dass** als oligomere Monocarbonsäuren (ii) solche auf Basis von Ölsäure, Ricinfettsäure und Tallölfettsäure ausgewählt sind, wobei Dimerfettsäuren auf Basis von Tallölfettsäuren besonders bevorzugt sind.

6. Ester nach mindestens einem der Ansprüche 1 bis 5, **dadurch gekennzeichnet, dass** die Ester eine kinematische Viskosität bei 40 °C im Bereich von größer 50 mm²/s bis 160 mm²/s und vorzugsweise von größer 100 mm²/s bis 160 mm²/s aufweisen.

7. Estermischung, enthaltend (a) mindestens einen Ester dessen Alkoholteil (iii) ausgewählt ist aus verzweigten primären Alkoholen mit mindestens 4 C-Atomen und dessen Säureteil (iv) ausgewählt ist aus verzweigten oder linearen Carbonsäuren mit 2 bis 22 C-Atomen und 2 bis 4 Carboxylgruppen, und (b) mindestens einem Ester gemäß den Ansprüchen 1 bis 6.

8. Estermischung nach Anspruch 7, **dadurch gekennzeichnet, dass** der Alkoholteil (iii) ausgewählt ist aus verzweigten Monoalkoholen mit 4 bis 44 C-Atomen, vorzugsweise 6 bis 24 C-Atomen und besonders bevorzugt mit 14 bis 24 C-Atomen.

9. Estermischung nach mindestens einem der Ansprüche 7 bis 8, **dadurch gekennzeichnet, dass** als Alkoholteil (iii) ein Guerbet-Alkohol mit 4 bis 44, vorzugsweise 6 bis 24, insbesondere 14 bis 24 und besonders bevorzugt mit 18 bis 22 C-Atomen ausgewählt ist.

10. Estermischung nach mindestens einem der Ansprüche 7 bis 9, **dadurch gekennzeichnet, dass** der Säureteil (iv) ausgewählt ist aus verzweigten oder unverzweigten Dicarbonsäuren und vorzugsweise aus linearen Dicarbonsäuren.

11. Estermischung nach mindestens einem der Ansprüche 7 bis 10, **dadurch gekennzeichnet, dass** der Säureteil (iv) ausgewählt ist aus der Gruppe Oxalsäure, Malonsäure, Bernsteinsäure, Glutarsäure, Adipinsäure, Pimelinsäure, Korksäure, Azelainsäure, Sebazinsäure, Maleinsäure, Fumarsäure und vorzugsweise aus Azelainsäure, Adipinsäure und Sebazinsäure, oder Mischungen dieser Säuren.

12. Estermischung nach mindestens einem der Ansprüche 7 bis 11, **dadurch gekennzeichnet, dass** die Komponenten (i) und (iii) gleiche Alkohole bzw. Alkoholmischungen sind.

13. Estermischung nach mindestens einem der Ansprüche 7 bis 12, **dadurch gekennzeichnet, dass** die Komponenten (i) und (iii) verschiedene Alkohole bzw. Alkoholmischungen sind.

14. Estermischung nach mindestens einem der Ansprüche 7 bis 13, **dadurch gekennzeichnet, dass** die Mischungen einen Viskositätsindex von mindesten 140, vorzugsweise größer/gleich 150 und insbesondere größer/gleich 160 aufweisen.

15. Estermischung nach mindestens einem der Ansprüche 7 bis 14, **dadurch gekennzeichnet, dass** die Mischungen einen Pourpoint von maximal - 35 °C, vorzugsweise maximal - 40 °C und insbesondere maximal - 45 °C aufweisen.

16. Estermischung nach mindestens einem der Ansprüche 7 bis 15, **dadurch gekennzeichnet, dass** die Estermischung einen Viskositätsindex von mindestens 50, einen Pourpoint von mindestens - 35 °C und einen kinematische Viskosität bei 40 °C von mehr als 50 mm²/s aufweist.

17. Verwendung eines Esters nach Anspruch 1 als Schmierstoff oder als Bestandteil einer Hydraulikflüssigkeit.

18. Verwendung einer Estermischung nach Anspruch 7 als Schmierstoff oder als Bestandteil einer Hydraulikflüssigkeit.
